# EUROPEAN PATENT SPECIFICATION

(11) **EP 4 466 046 B1**
(45) Date of publication and mention of the grant of the patent: **16.04.2025**
(21) Application number: 23771926.5
(22) Date of filing: 06.09.2023
(51) Int. Cl.: A61M 15/00

(54) **DRY POWDER INHALER**
TROCKENPULVERINHALATOR
INHALATEUR DE POUDRE SÈCHE

(30) Priority: 14.09.2022 DE 102022123400
(43) Date of publication of application: 27.11.2024
(73) Proprietor: Emphasys Importadora Exportadora e Distribuidora Ltda., 18540-000 Porto Feliz SP (BR)
(72) Inventor: ESTEVE, Victor, Itú, SP 13.306-530 (BR)
(74) Representative: DREISS Patentanwälte PartG mbB
(86) International application number: PCT/IB2023/000489
(87) International publication number: WO 2024/057086

(56) References cited:
- EP-A1- 3 138 601
- EP-A2- 1 270 034
- WO-A1-2018/115915
- US-A1- 2013 276 783
- US-A1- 2015 231 344
- US-A1- 2018 200 460

## Description

The invention relates to a dry powder inhaler for a capsule containing dry powder.

Such a dry powder inhaler is known, for example from EP 1 270 034 A1 or from US 2003/0000523. Those dry powder inhalers are typically non-pressurized inhalers. A capsule that is arranged in the capsule receptacle of the dry powder inhaler can be pierced by the needles. When air is sucked in by a patient through a mouthpiece, the capsule is lifted from the capsule receptacle into a capsule chamber and rotates in the capsule chamber. Upon rotation of the capsule, dry powder contained in the capsule is dispersed by centrifugal forces during rotation and mixed with air such that it can be inhaled by a patient.

WO 2018/115915 A1 discloses a dry powder inhaler for a capsule containing dry powder. The inhaler comprises a housing having a capsule receptacle, two actuator buttons arranged on opposing sides of the housing and two perforation needles, each needle being fixedly connected to an actuator button and movable relative to the housing towards each other from a normal position to a perforation position along an actuation direction to perforate a capsule arranged in the capsule receptacle, wherein a first end of each actuator button is connected to the housing at a lower portion of the housing and in that a second free end of each actuator button is movable into the perforation position such that the perforation needle is moved along a circular line.

EP 3 138 601 A1 discloses a dry powder inhaler for a capsule containing dry powder. The inhaler comprises a housing having a capsule receptacle, two actuator buttons arranged on opposing sides of the housing and two perforation needles, each needle being fixedly connected to an actuator button and movable relative to the housing towards each other from a normal position to a perforation position along an actuation direction to perforate a capsule arranged in the capsule receptacle, wherein a first end of each actuator button is connected to the housing at a lower portion of the housing and in that a second free end of each actuator button is movable into the perforation position such that the perforation needle is moved along a circular line.

In order to disperse the dry powder contained in the capsule, the capsule has to be perforated by the needles.

The problem of the known mono-dose dry powder inhalers is that the dosage of powder is limited. When piercing the capsule directly in its hemispherical ends, the amount of powder to be dispersed during inhalation is limited because the flow of the dry powder out of the pierced openings is obstructed during rotational movement of the capsule due to the arrangement of the openings. The obstruction can be caused by cut-out portions of the capsule shell are bended inwards by the needles when piercing the capsule. Moreover, the known dry powder inhalers are limited in the maximum dosage of dry powder. In certain areas of application a high dosage in the range of about 25 to about 200 mg of dry powder in one or multiple capsules is required such as in treatments or clinical tests for cystic fibrosis, tuberculosis, pulmonary artery hypertension, neurologic and congenital disorders, Parkinson, asthma, Chronic obstructive pulmonary disease (COPD) etc.

Moreover, known inhalers often have the problem that a wide variation in the inhalation resistance can occur depending on the pulmonary capacity of a user or patient, wherein a high inhalation resistance - at a typical pulmonary capacity of about 60 l/min - should preferably be avoided. It is therefore an object of the present invention to provide a cost effective dry powder inhaler for dispersion of dry powder, which provides for easy handling of large dosages of dry powder without a high inhalation resistance.

This object is solved by a dry powder inhaler according to the features of claim 1. Preferred embodiments are matter of the dependent claims.

An aspect of the description is directed to the following subject-matter: A dry powder inhaler for a capsule containing dry powder, the inhaler comprising a base body having a capsule receptacle, two actuator buttons arranged on opposing sides of the base body and two perforation needles, each needle being fixedly connected to a respective one of the actuator buttons and movable relative to guiding sections of the base body towards each other from a normal position to a perforation position along an actuation direction to perforate a capsule arranged in the capsule receptacle, wherein the capsule receptacle is arranged in an inclined angle within the range of about 40° to about 50° with respect to the actuation direction, characterized in that an extra-centric tip of at least one of the piercing needle is facing the longitudinal axis of the other piercing needle.

When using the dry powder inhaler, the extra-centric tip and its arrangement result in a larger and triangular-shaped opening when piercing the capsule. This makes it easier to expel the powder contained in the capsule. The air-powder mixture inhaled by the patient can be improved in this way. As a result of the fact that larger quantities of powder are safely discharged from the capsule, a reduction of emitted dose variaton is achieved and thus the drug can be delivered more precisely. Therefore, exact dosing of drugs is improved.

Moreover, the piercing method results in more homogeneous shaping and size of the piercing holes. Accordingly, a homogeneity between different doses of the drug is achieved compared to other forms of capsule piercing.

The cutted shape of the opening prevents the closing of the inwards portion of the capsule due to its unique triangular-like shape with two lateral parts bended inwards. This has to do with the needle-tip shape and a contact direction with the capsule.

A beneficial example is characterized by that the extracentric tip of both the piercing needles face the longitudinal axis of the other piercing needle.

Another example is characterized by that the extracentric tip of at least one of the perforation needles passes an imaginary plane, which is tangential to an associated curved section of the capsule receptacle, with an angle between 90° and 83°, in particular between 90° and 85°, in particular between 90° and 88°, when being moved between the normal position to the perforation position along the actuation direction.

This nearly perpendicular movement of the needle tip through the imaginary plane causes the needle tip to enter the capsule shell nearly perpendicularly. By this piercing arrangement, the hole in the capsule will be enlarged.

Another example is characterized by that a central longitudinal axis of the guiding section enters a distal portion of the curved section in the capsule receptacle.

This arrangement causes the needle to enter in a distal portion of the associated capsule hemisphere. Therefore, the hole generated in the capsule is at a distal point easing the way for the powder to be released from the inside of the capsule.

For example, benefits can be achieved by that at least one of the guiding sections and the associated needle are arranged such that the tip of at least one of the needles enters the capsule receptacle in a distal portion of a curved section of the capsule receptacle.

Going further, an example is advantageous by that the guiding sections of the base body extend in imaginary parallel planes.

According to an example of the inhaler, the first end of each actuator button is pivotally attached to the housing.

It is particularly preferred if the first end of each actuator button is connected to the housing via a ball joint snap-on connection pivoting around a pivot axis. By providing a ball joint snap-on connection, the actuator buttons can be attached to the lower portion of the housing by engaging the ball point snap-on connection.

Advantageously, the actuator buttons are pivotable around a pivoting angle of about 5 to 15°, preferably of about 10°.

In another preferred example of the inhaler, the ball joint snap-on connection comprises hemi-spherical protrusions arranged on the housing and hemi-spherical recesses arranged on the actuator buttons complementary to the hemispherical protrusions, wherein the protrusions and recesses extend along the pivot axis. In order to engage the ball joint snap-on connection, the hemi-spherical protrusions are preferably arranged on the housing such that they can be elastically pushed into a release position along the direction of the pivot axis. In the release position, the recesses of the actuator buttons and the protrusions of the housing can be superimposed such that the protrusions can engage the recesses and elastically move back into an engagement position of the ball joint snap-on connection.

It is particularly preferred if the perforation needles are arranged in an area of the second free end of each actuator button spaced away from the first end of the actuator button. Preferably, the perforation needles are arranged on the inside surfaces of the actuator buttons facing each other. By arranging the perforation needles in an area of the second free end of each actuator button, a large movement range can be provided despite connecting a first end of each actuator button to the housing.

In a particularly preferred example, the dry powder inhaler comprises a spring element acting upon the actuator buttons such that the actuator buttons are pre-loaded into the normal position. Preferably, the spring element is a helical spring.

In another preferred example of the dry powder inhaler, the housing comprises two openings configured to receive the actuator buttons such that an outside surface of the housing and the actuator buttons is substantially flush when the actuator buttons are in the normal position. It is particularly preferred if the actuator buttons in particular their flaps on the laterals close the openings in the housing in the normal position in a substantially airtight manner such that no or substantially no air can pass between the actuator buttons and the opening edges.

The dry powder inhaler is characterized in that an intermediate air chamber is formed between the base body and the covering body, wherein the mouthpiece has an inhaling inlet fluidly connected to the capsule chamber and at least one mouthpiece air inlet arranged on a lateral outside of the mouthpiece and fluidly connected to the intermediate air chamber and wherein the base body has at least one base body air inlet being fluidly connected to the capsule chamber and to the intermediate air chamber and ending in the capsule chamber.

Preferably, the base body is connected to the covering body such that no or substantially no air can be sucked out of the capsule chamber at the joint surface between the base body and the covering body. It is particularly preferred if two actuator buttons are provided on opposing sides of the covering body movable between a normal position and a perforation position. Advantageously, the actuator buttons are closing the covering body in the normal position in a substantially airtight manner such that no or substantially no air can pass between the actuator buttons and opening edges of the openings which are configured to receive the actuator buttons. It is therefore particularly preferred that substantially no air can be sucked in between the covering body and the actuator buttons when the actuator buttons are in the normal position. In a preferred embodiment of the dry powder inhaler the at least one base body air inlet ends tangentially into the capsule chamber. Preferably, two base body air inlets are provided. This provides a more stable resistance to air flow during inhalation as no quanity of air stream can erraticly enter the housing chamber during inhalation.

When a patient or user is using the dry powder inhaler, a capsule arranged in the capsule chamber can be pierced by moving the actuator buttons from the normal position into the perforation position along an actuation direction. After piercing the capsule, the actuator buttons are moved back into the normal position. When a patient or user is sucking in air through the inhaling inlet of the mouthpiece, a partial vacuum in the capsule chamber can be generated wherein air is sucked through the capsule chamber through the at least one base body air inlet generating an air flow that lifts the capsule in the capsule chamber and rotates the capsule in the capsule chamber such that dry powder contained in the capsule chamber can be dispersed.

Advantageously, the air sucked through the capsule chamber through the at least one base body air inlet can be directed from the mouthpiece air inlet and through the intermediate air chamber. By directing the airflow through the intermediate air chamber, a dry powder inhaler can be provided with less necessary pressure for achieving a certain average flow rate.

According to a preferred example of the dry powder inhaler, the capsule chamber is circular cylindrical and comprises a hemi-spherical end-section having a capsule receptacle. The capsule receptacle can be arranged in an angle with respect to an actuation direction of the actuator buttons. Nevertheless, it is also possible that the capsule receptacle is arranged parallel to the actuation direction.

Preferably, the mouthpiece comprises a cylindrical protrusion configured to extend at least partially into the capsule chamber.

In a particularly preferred example of the dry powder inhaler, the cylindrical protrusion of the mouthpiece extends into the capsule chamber wherein a lateral surface of the cylindrical protrusion is configured to sealingly abut on an inside surface of the capsule chamber. Preferably, the cylindrical protrusion abuts such the inside surface of the capsule chamber that substantially no air can be sucked out of the capsule chamber between the inside surface of the capsule chamber and the lateral surface of the cylindrical protrusion.

In another preferred example of the dry powder inhaler, the base body comprises at least one base body duct overlapping with and in fluid connection with the mouthpiece air inlet, wherein the base body duct is fluidly connected to the intermediate air chamber and ends in the intermediate air chamber. Preferably, the base body duct is an opening in the base body overlapping with the mouthpiece air inlet.

Preferably, the base body comprises a cylindrical shoulder which is preferably oval and a base body plate which is preferably oval and arranged perpendicular to the cylindrical shoulder. Preferably, the base body plate defines an outside wall of the intermediate air chamber. Advantageously, the at least one base body duct is an opening in the base body plate overlapping with the mouthpiece air inlet such that the intermediate air chamber is fluidly connected to the mouthpiece air inlet.

In a preferred example of the dry powder inhaler, the capsule chamber is attached to the base body plate and opens into the base body plate.

According to an advantageous example, the respective actuator button is pivotable around a pivot axis with a pivoting angle, for example of about 5° to 15°, preferably of about 10°, wherein the respective actuator button comprises a countour moving along a circular path around the pivot axis, and wherein the countour engages in a guiding countour of the base body.

Advantageously, guiding means are provided to obtain piercing results that are stable and repetitive with regard to the realeased powder.

According to an advantageous example, the actuator button comprises a sealing surface that surrounds at least partly an outward actuator surface, and wherein the contour that engages in the guiding contour of the base body is arranged proximal to or flush to the outwardly facing sealing surface of the respective actuator button.

Advantageously, the functions of guiding and sealing are seperated.

Further details and advantages of the invention can be taken from the following description, on the basis of which the embodiments of the invention that are represented in the Figures are described and explained in more detail.

Respective figures are showing:
Figure 1 a side view of a dry powder inhaler according to the invention;
Figure 2 a perspective view of the dry powder inhaler of Figure 1;
Figure 3 a sectional view of a part of the dry powder inhaler of Figures 1 and 2;
Figure 4 a perspective view showing parts of the dry powder inhaler of Figures 1 to 3;
Figure 5 a perspective view of a base body of the dry powder inhaler of Figures 1 to 4;
Figure 6 a perspective top view of the base body of Figure 5;
Figure 7 a top perspective view of a dry powder inhaler of Figures 1 to 4 in an open position;
Figure 8 a top view of the base body of Figures 5 and 6;
Figure 9 a capsule and two needles in a piercing position;
Figure 10 a side view of a needle of the dry powder inhaler of Figures 1 to 4;
Figure 11 a top view of the needle of Figure 10;
Figure 12 a front view of the needle of Figures 10 and 11;
Figure 13 a schematic view of a pierced capsule during inhalation;
Figure 14 a comparison of the achieved piercing result with the prior art results;
Figure 15 a partly sectional view of the dry powder inhaler;
Figure 16 a close-up view of figure 15;
Figure 17 a side view of the dry powder inhaler;
Figure 18 a close-up view of figure 17; and
Figure 19 a view of the base body.

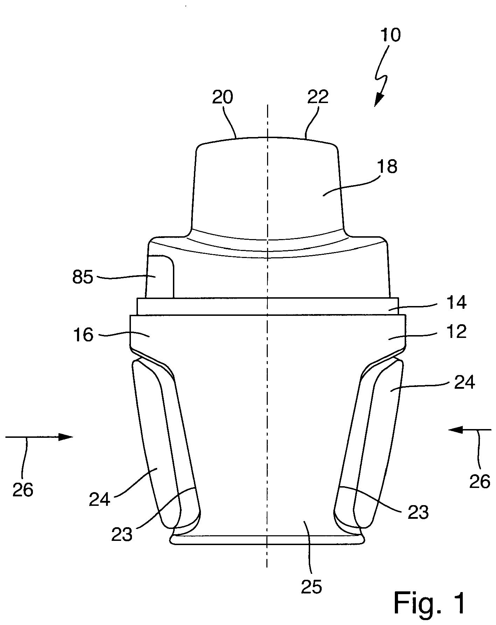

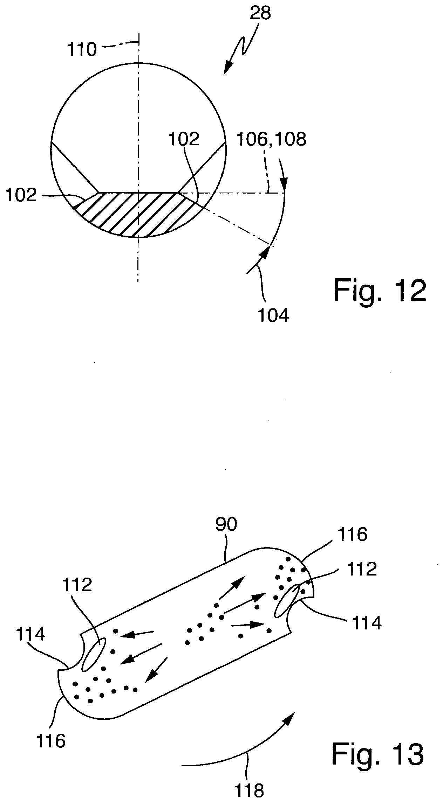

Figures 1 to 12 show a dry powder inhaler 10 for a capsule containing dry powder (shown in Figure 13). The dry powder inhaler 10 comprises a housing 12 having a base body 14 and a covering body 16. The dry powder inhaler 10 furthermore comprises a mouthpiece 18. The mouthpiece 18 has a mouth portion 20 having a central opening 22 for inhaling air that mixes with dry powder contained in a capsule arranged in the dry powder inhaler 10.

The dry powder inhaler 10 comprises two actuator buttons 24 arranged on opposing sides of the housing 12 which are movable relative to the housing 12 towards each other along an actuation direction which is indicated by arrows 26 from a normal position to a perforation position to perforate a capsule arranged in the dry powder inhaler 10. The housing 12 comprises two openings 23 configured to receive the actuator buttons 24 such than an outside surface 25 of the housing 12 and the actuator buttons 24 is substantially flush when the actuator buttons 24 are in the normal position.

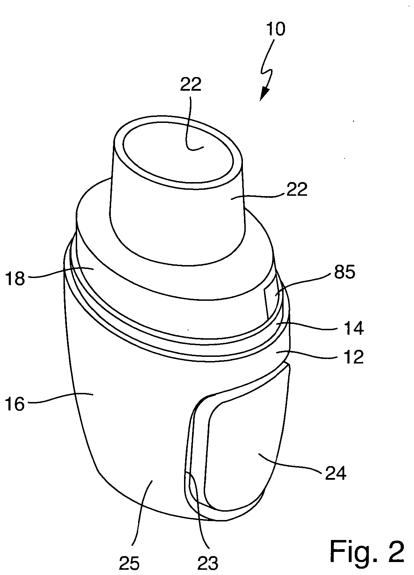

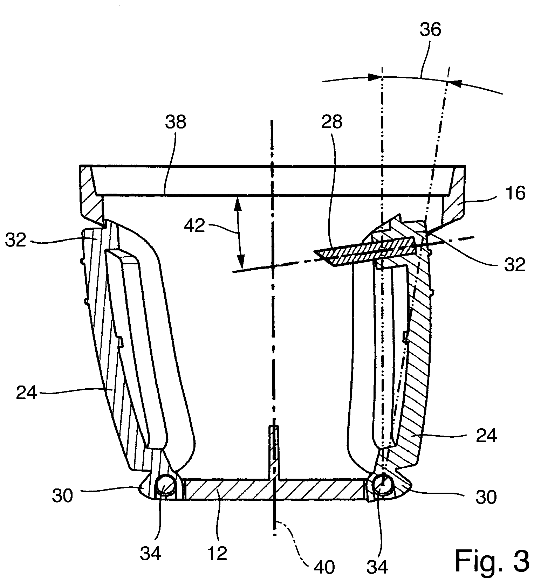

Figure 3 shows a sectional view of a part of the dry powder inhaler 10 of Figures 1 and 2. Each actuator button 24 is attached the covering body 16, i.e. the housing 12, wherein a perforation needle 28 is provided being fixedly connected to an actuator button 24. Figure 3 shows only one perforation needle 28 because the needles 28 are arranged with an offset concerning the cutting plane of Figure 3.

A first end 30 of each actuator button 24 is connected to the housing 12, i.e. the covering body 16 in a lower portion of the housing 12. The perforation needles 28 are arranged in an area of a second free end 32 of the actuator buttons 24. This second free end 32 is spaced away from the first end 30. The second free end 32 of the actuator buttons 24 is movable from the normal position into the perforation position shown in Figure 3 such that a tip of the perforation needle 28 is moved along a circular line.

The first end 30 of each actuator button 24 is pivotably attached to the housing 12 by use of a ball joint snap-on connection 34 pivoting around a pivot axis, wherein the actuator button is pivotable around a pivoting angle 36 of about 5° to 15°, preferably of about 10°. Accordingly, the perforation needles 28 are inclined with respect to a plane 38 which is perpendicular to a longitudinal axis 40 of the dry powder inhaler 10 in a perforation angle 42 of about 5° to 15°, preferably of about 10° when the actuator buttons 24 are in the perforation position.

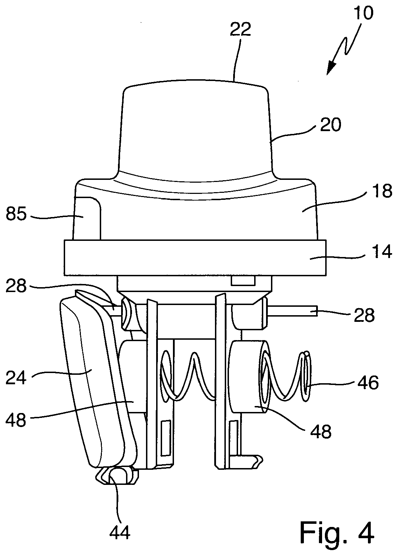

The ball joint snap-on connection 34 comprises hemispherical protrusions (not shown in the drawings) arranged on the housing 12 and hemi-spherical recesses 44 (shown in Figure 4) arranged on the actuator buttons 24, wherein the protrusions and recesses extend along the pivot axis and wherein the protrusions engage the recesses.

By arranging the two actuator buttons 24 on opposing sides of the housing 12 and connecting each actuator button 24 to the housing 12 at a lower portion of the housing 12, the actuator buttons 24 can be pre-loaded into the normal position by use of only one spring element.

Such a spring element is shown in Figure 4 which shows a perspective view with of parts of the dry powder inhaler. The dry powder inhaler 10 comprises a helical spring 46 arranged in shell-like spring-guiding members 48 of the base body 12 and acting upon the actuator buttons 24 such that the actuator buttons 24 are pre-loaded into the normal position against the direction depicted by arrows 26.

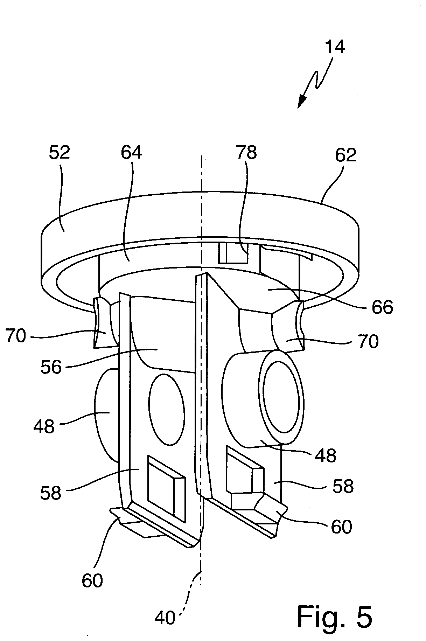

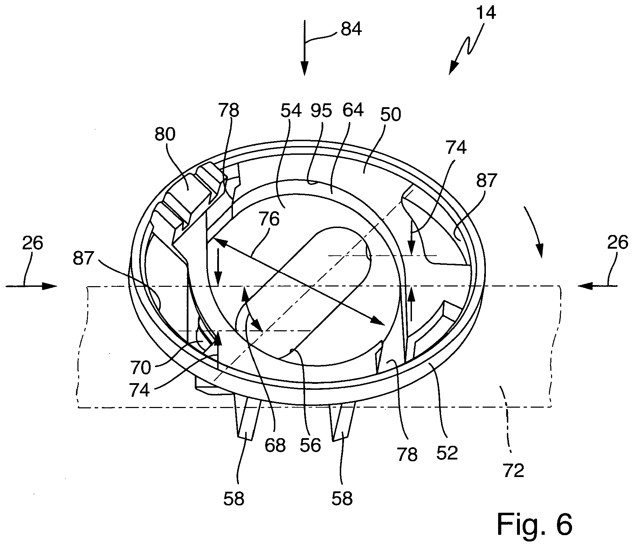

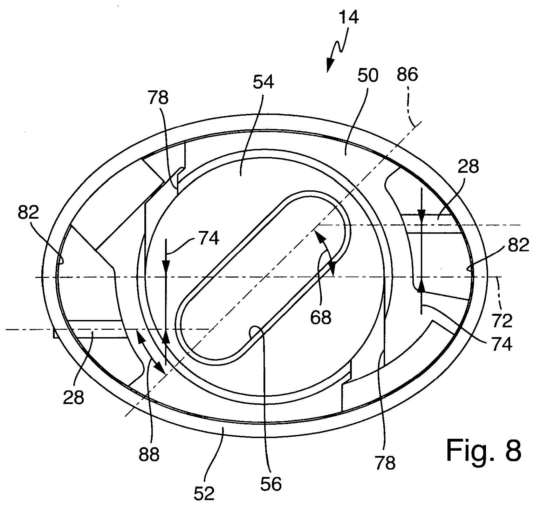

The base body 14 of the dry powder inhaler 10 which is made of one piece is shown in more detail in Figures 5 and 6. Figure 8 furthermore shows a top view of the base body 14 of Figures 5 and 6. The base body 14 has an elliptical or oval base body plate 50 and an oval cylindrical shoulder 52 extending perpendicular to the base body plate 50 and connected to the base body plate 50. The base body 14 has a capsule chamber 54 having a capsule receptacle 56 for receiving a dry powder capsule and two downwardly extending plate members 58 that are substantially parallel to each other. The spring-guiding members 48 are arranged coaxially to the actuation direction 26 wherein the plate members 58 are arranged perpendicular to the actuation direction 26. At a free end of the plate members 58, bearing sections 60 for the actuator buttons 24 are provided.

The capsule chamber 54 is open towards an upper end portion 62 of the base body 14. The capsule chamber 54 has a circular cylindrical section 64 and a hemi-spherical or conical end section 66, wherein the capsule receptacle 56 is arranged in the hemi-spherical end section 66 of the capsule chamber 54. The capsule chamber 54 has, for example, a volume of about 2500 to about 3000 mm³ or less. The capsule receptacle 56 is arranged perpendicular to the longitudinal axis 40 of the dry powder inhaler 10.

The capsule receptacle 56 is furthermore arranged in an inclined angle 68 within the range of 40° to 50° with respect to the actuation direction 26. The base body 14 comprises two hollow needle guiding sections 70 configured to guide the needles 28 and being arranged perpendicular to the longitudinal axis 40 of the inhaler 10 and ending in the capsule receptacle 56. Those needle guiding sections 70 and the needles 28 are arranged parallel to the actuation direction 26 and spaced apart from a middle plane 72 of the base body 14 in a distance 74. The needles 28 are arranged on opposing sides of the middle plane 72.

According to another embodiment not shown in the drawings and not claimed, the capsule receptacle 56 is not arranged in an inclined angle with respect to the actuation direction 26 but is parallel to the actuation direction 26. In that case the needle guiding sections 70 and the needles 28 are arranged parallel to the actuation direction 26 but are not spaced apart from the middle plane 72 of the base body 14. With this embodiment, a capsule arranged in the capsule receptacle 56 can be pierced directly in the hemi-spherical end-section of the capsule.

The circular cylindrical section 64 has a diameter 76 which is bigger than the length of the capsule receptacle 56. The size of the capsule receptacle 56 corresponds with the size of the capsule such that the capsule receptacle 56 is only slightly bigger than the capsule to ensure that the capsule is held in the capsule receptacle 56 when pushing the actuator buttons 24 and piercing the capsule with the needles 28. Preferably, the capsule receptacle 56 is configured to receive a capsule with a volume in the range of about 400 to about 500 mm³.

The base body 14 furthermore has two base body air inlets 78 in the area of the cylindrical shoulder 52 which are ending tangentially into the capsule chamber 54 and are arranged perpendicular to the middle plane 72 of the base body 14. The mouthpiece 18 is fastened to the base body 14 via a hinge. A lower part of the hinge is depicted in Figure 6 and having the reference numeral 80. The base body 14 also comprises two base body ducts 82.

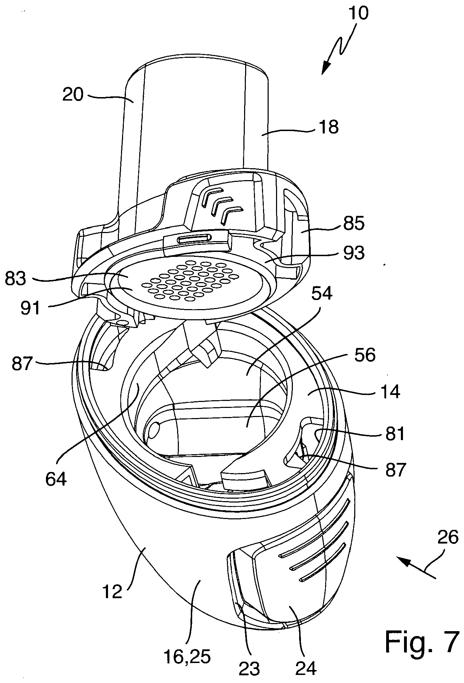

Between the base body 14 and the covering body 16 of the housing 12 an intermediate air chamber 81 is formed. The central opening 22 of the mouthpiece 18 functions as inhaling inlet and is fluidly connected to the capsule chamber 54 via a perforated plate 83 as shown in Figure 7. The mouthpiece 18 has two mouthpiece air inlets 85 on a lateral outside of the mouthpiece which are fluidly connected to the intermediate air chamber 81.

The base body air inlets 78 are also fluidly connected to the intermediate air chamber 81 and to the capsule chamber 54. The base body comprises two base body ducts 87 overlapping with and in fluid connection with the mouthpiece air inlets 85. The base body ducts 87 are also fluidly connected to the intermediate air chamber 81 and end in the intermediate air chamber 81.

On the side facing away from the mouth portion 20, the mouthpiece 18 comprises a cylindrical protrusion 91 configured to extend at least partially into the capsule chamber 54. The perforated plate 83 is arranged in an end-section of the cylindrical protrusion 91. A lateral surface 93 of the cylindrical protrusion 91 is configured to sealingly abut on an inside surface 95 of the capsule chamber 54 such that substantially no air can pass between the inside surface 95 of the capsule chamber 54 and the lateral surface 93 of the cylindrical protrusion.

Figure 8 shows a top view of the base body 14 of Figure 6 when viewing in the direction of arrow 84 in Figure 6. The capsule receptacle 56 has a longitudinal axis 86 which is arranged in an inclined angle 68 within the range of about 40° to about 50° with respect to the actuation direction 26 or the middle plane 72. The needles 28 are piercing a capsule which can be arranged in the capsule receptacle 56 in an angle 88 with respect to the longitudinal axis 86 of the capsule receptacle 56 or the capsule.

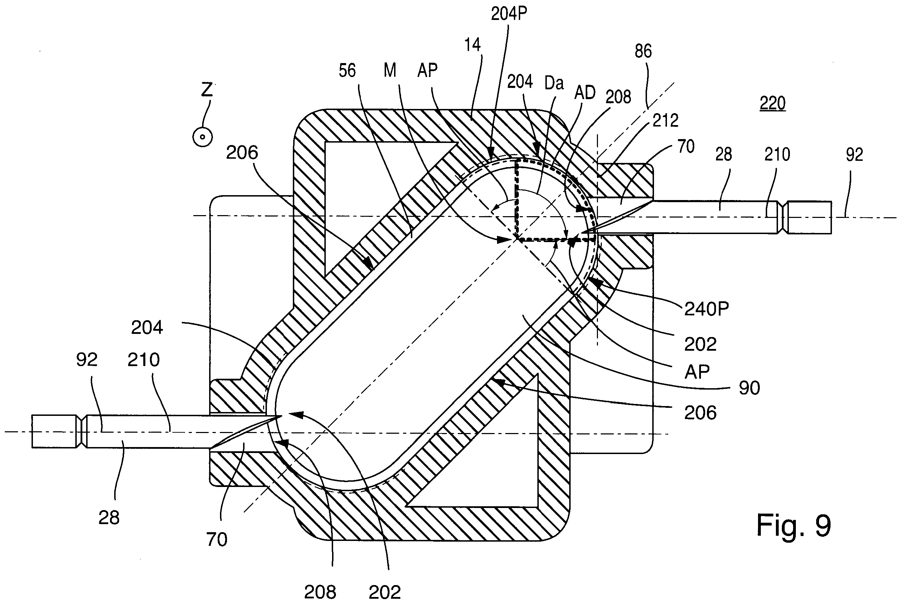

Such a capsule 90 is shown in Figure 9. The longitudinal axis 86 of the capsule 90 is arranged to a longitudinal axis 92 of the needles 28 in the angle 88.

Figure 9 depicts a schematical cross section of the base body 14 along the middle axes of the guiding sections 70.

The figure concerns a dry powder inhaler 10 for a capsule 90 containing dry powder, the inhaler 10 comprising a base body 14 having a capsule receptacle 56, two actuator buttons 24 arranged on opposing sides of the base body 14 and two perforation needles 28, each needle 28 being fixedly connected to a respective one of the actuator buttons 24 and movable relative to guiding sections 70 of the base body 14 towards each other from a normal position to a perforation position along an actuation direction 26 to perforate a capsule 90 arranged in the capsule receptacle 56, wherein the capsule receptacle 56 is arranged in an inclined angle 68 within the range of about 40° to about 50° with respect to the actuation direction 26, characterized in that an extracentric tip 202 of at least one of the piercing needle 28 is facing the longitudinal axis 92 of the other piercing needle 28.

There is depicted that the extracentric tip 202 of both the piercing needles 28 face the longitudinal axis 92 of the other piercing needle 28.

Shown is that the extracentric tip 202 of at least one of the perforation needles 28 passes an imaginary plane 212, which is tangential to an associated curved section 204 of the capsule receptacle 56, with an angle between 90° and 83°, in particular between 90° and 85°, in particular between 90° and 88°, when being moved between the normal position to the perforation position along the actuation direction 26. In other words, the axis of movement of the perforation needle 28 passes the imaginary plane 210 perpendicularly or nearly perpendicularly.

In the section shown perpendicular to the vertical axis z, the imaginary plane 210 abuts the section 204 and passes through an edge of the opening 208, wherein said edge faces the axis 210 of the opposing guiding section 70.

Shown is that a central longitudinal axis 204 of the guiding section 210 enters a distal portion 204D of the curved section 204 in the capsule receptacle 56.

The distal region 204D of the curved section 204 differs from the proximal regions 204P in that the distal region 240D of the curvature are disposed along the longitudinal extent according to the axis 86 away from the center wall 206, whereas the proximal regions 204P of the curvature are disposed between the wall 206 and the distal region 204D.

An imaginary plane 220, which corresponds to the cross-sectional plane in Figure 9, is defined by the two central longitudinal axes 210. In the imaginary plane 220, the distal region 240D comprises an arc of the semicircle of the curved section 204 with an angle AD of about 90° starting from an imaginary center point M. The proximal regions 204P arranged on both sides of the distal region 204D comprise a respective angle AP of about 45° starting from the imaginary center point M. The former aspect can also be applied to the capsule 90 to be opened.

The needle tip 202 of the respective needle 28 is arranged in such a way that it contacts the shell of the capsule 90 in its distal region of the hemisphere. In line with the current invention, the needle tip 102 is arranged extracentrically and is located on an imaginary cylindrical barrel of the needle 28. The present rotational position of the needle 28 determines the needle tip 102 in a tip position which faces the other axis 92 of the opposite guide section 70. This arrangement ensures that the needle tip 202 hits the capsule shell substantially perpendicularly. The more perpendicular the impact direction, the larger the opening in the capsule 90. Nonetheless, the opening in the shell of the capsule 90 is created in a distal region of the surface of the hemisphere, the distal region of the hemisphere including an angle of 90° to the center M surrounded by a proximal region of the hemisphere, the proximal region of the hemisphere being arranged in a cross-section lateral to the proximal region and each including an angle of 45° to the center M.

There is illustrated that at least one of the guiding sections 70 and the associated needle 28 are arranged such that the tip 202 of at least one of the needles 28 enters the capsule receptacle 56 in a distal portion 204D of a curved section 204 of the capsule receptacle 56.

The figure is directed to an example in that the guiding sections 70 of the base body 14 extend in parallel imaginary planes.

The capsule receptacle 56 is delimited by two distal curved sections 204, each of which follows a semicircle in perpendicular cross-section to the vertical axis z of the dry powder inhaler 10. The curved sections 204 follow a hemisphere at least partly. The two curved sections 204 are connected to each other by a wall 206 extending parallel to the axis 86. The sections 204 and the wall 206 form the capsule receptacle 56. The guiding sections 70 enter the capsule receptacle 56 via an opening 208. A longitudinal axis 210 of the guiding section 70 coincides with the axis 92 of the associated needle 28.

The piercing needles are rotated 90° left with respect to the axis 210 in relation to the prior art.

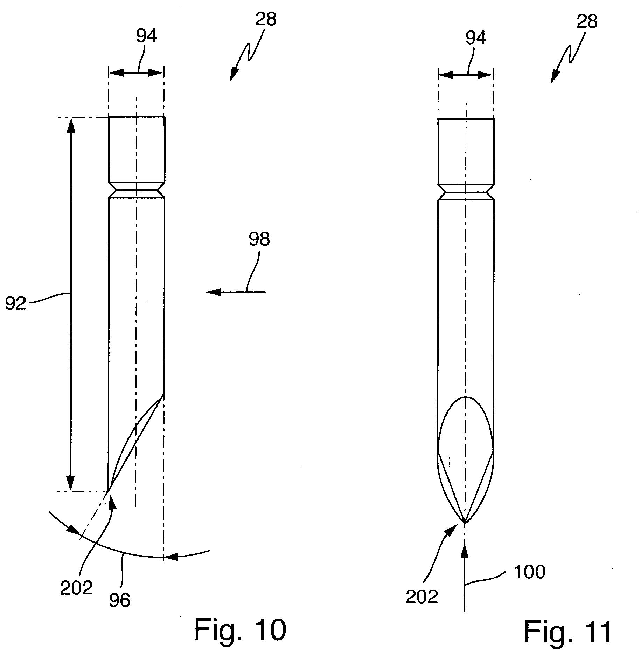

Figure 10 depicts a side view of one of the needles 28 of the dry powder inhaler 10. The needles 28 have a length 92, a needle diameter 94 and a cutting tip angle 96. The needle diameter 94 is in the range of about 1,10 to about 1,95 mm wherein the cutting tip angle 96 is in the range of about 20° to 40°. A distal part comprises a bevel following the angle 96. Said bevel includes two lateral chamferings meeting at the tip 202 to improve the sharpness of the tip 202. The respective distal part of the needle 28 is bevel-like or lancet-like.

Figure 11 shows a top view of the needle 28 of Figure 10 when looking into the direction of arrow 98 in Figure 10.

Figure 12 shows a partial cross-section A-A of the needle 28 of Figures 10 and 11 in a front view when looking into the direction of arrow 100 in Figure 11.

The needles 28 have lateral cutting edges 102 which are arranged in an angle 104 in the range of about 25 to 35° with respect to an axis 106 perpendicular to the longitudinal axis 92 of the needle 28 and lying in a plane 108 parallel to the longitudinal axis 92. The needles 28 are symmetrical to a middle plane 110.

The dry powder inhaler 10 functions as follows:
In order to insert a dry powder capsule 90 into the capsule receptacle 56 of the base body 14, the mouthpiece 18 can be pivoted into an opening position via the hinge 80 as shown in Figure 7. After insertion of the capsule 90, the mouthpiece 18 is pivoted back into a closed position as depicted in Figures 1 and 2. In order to pierce the capsule 90, the actuator buttons 24 are pressed against the force of the helical spring 46 into the actuation direction 26.

The cutting tip angle 96, the lateral cutting edges 102 and the arrangement of the capsule receptacle 56 and the needles 28 provide for an accurate cutting of a shell of the capsule 90 during piercing of the capsule 90 without detaching the cut-out portion from the capsule shell.

Figure 13 depicts a schematic view of a pierced capsule 90 during inhalation. Cut-out portions 112 can be bent inwards into the interior of the capsule 90 in a hinged or flap-like manner.

Because of the comparably large needle diameter 94, comparably big openings 114 can be pierced into the capsule 90 in a transition area of the hemispherical ends 116 and the tubular middle section of the capsule 90.

When a patient or user is using the dry powder inhaler 10, a capsule 90 arranged in the capsule receptacle 56 can be pierced by moving the actuator buttons 24 from the normal position into the perforation position. After piercing the capsule 90, the actuator buttons 24 are moved back into the normal position. When a patient or user is sucking in air through the inhaling inlet 22 of the mouthpiece 18, a partial vacuum in the capsule chamber 54 can be generated wherein air is sucked into the capsule chamber 54 through the at least one base body air inlet 78 generating an air flow that lifts the capsule 90 from the capsule receptacle 56 into the capsule chamber 54 and rotates the capsule 90 in the capsule chamber 54 in the direction of arrow 118 as shown in Figure 13 such that dry powder contained in the capsule 90 can be dispersed.

When sucking in air on the inhaling inlet 22 through the capsule chamber 54, air is directed from the mouthpiece air inlets 85 via the base body ducts 87 and through the intermediate air chamber 81 into the capsule chamber 54 via the base body air inlets 78.

Table 1 and Table 2 shown below show comparative tests of flow resistances of a dry powder inhaler 10 having an intermediate air chamber 81 and flow resistances of a dry powder inhaler without an intermediate air chamber 81.

**Table 1: flow resistance at different air flow**

| inhaler with intermediate air chamber / air buffer | | |
|---|---|---|
| L/min, | Value | Unit |
| 30 | 0,74 | kPA |
| **60** | **1,56** | **kPA** |
| 90 | 3,52 | kPA |
| 100 | 3, 81 | kPA |

**Table 2: flow resistance at different air flow**

| inhaler without intermediate air chamber | | |
|---|---|---|
| L/min, | Value | Unit |
| 30 | 0, 82 | kPA |
| **60** | **2,16** | **kPA** |
| 90 | 3, 83 | kPA |
| 100 | 4, 02 | kPA |

By directing the air flow through the intermediate air chamber 81, a dry powder inhaler 10 can be provided with less necessary pressure for achieving a certain average flow rate in contrast to a dry powder inhaler which has no intermediate air chamber 81.

Overall, an inhaler 10 with an intermediate air chamber 81 can be provided that has less flow resistance at regular lung capacities. When a user or patient has less lung capacity of about 60L/min, a dry powder inhaler 10 can be provided with significantly less flow resistance in comparison to a dry powder inhaler without an intermediate air chamber 81.

Because of the arrangement of the capsule receptacle 56 and the needles 28, the capsule 90 can be pierced in a position that allows powder to be efficiently dispersed during the rotational movement in an inhalation process while providing little or almost no obstruction to the powder flow.

To sum up, a dry powder inhaler 10 is provided which allows for a high dosage inhalation in the range of about 25 to about 200 mg.

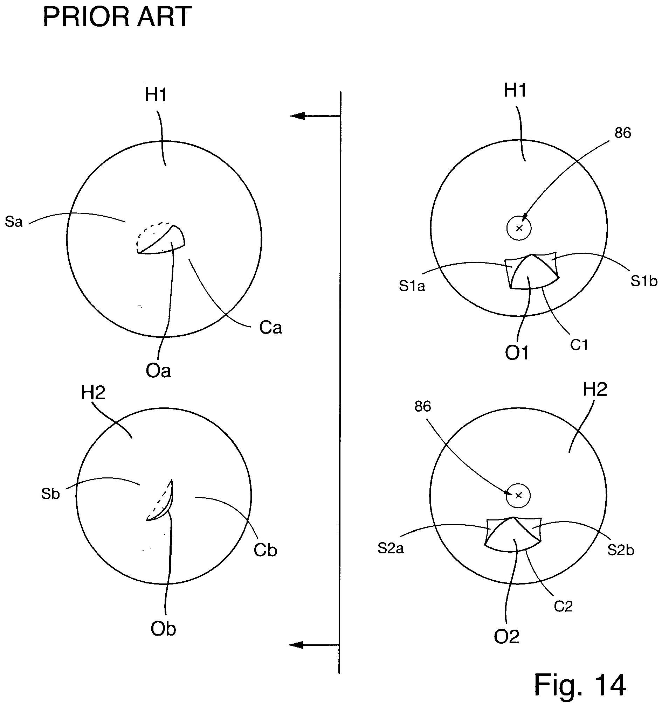

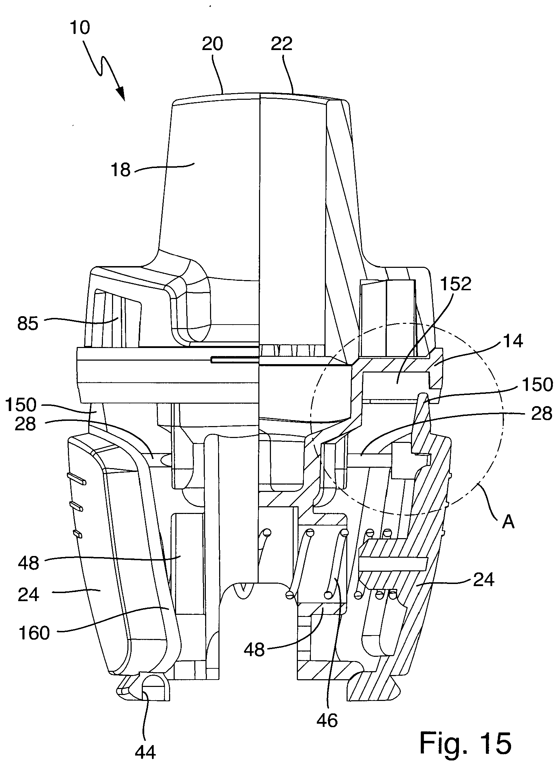

Figure 14 shows internal images of capsule hollow mold portions H1, H2 which can be inserted into one another and which, in the right-hand illustration, have been opened using the dry powder inhaler according to the embodiment shown in Figure 7. That is the views of figure 15 show the internal wall of the hemispherical part of the hollow molds H1, H2.

For comparison purposes, two capsule halves are shown on the left which were opened using a dry powder inhaler according to prior art. The hard capsules consist of two prefabricated cylindrically shaped hollow mold parts H1, H2 with hemispherical ends. In the images shown, perforations have been introduced into both of the hemispherical ends by piercing with a respective needle. It can be clearly seen that the capsule parts shown on the right have larger perforations O1, O2 than the openings Oa, Ob which are achievable by a piercing method according to the prior art. This results in one small and strong flap per per mold part H1, H2, which can be bended back outwards during inhalation preventing the powder to easily exit the capsule. Larger flaps tend to return to its original position of the shell in case of large portions of powder wheight inside the capsule.

In the prior art piercing method, only a single segment Sa, Sb of the hollow molds is created by piercing the capsule hemispheres along a continuous crack border Ca, Cb. The single segment Sa, Sb per hemisphere projects inwards with respect to the shell of the capsule.

The piercing method described in this description provides, in contrast to the prior art method, that the respective piercing needle creates two segments S1a, S1b and S2a, S2b of the shell per hemisphere. Both segments S1a, S1b and S2a, S2b per hemisphere.

The two fold edges of the flaps or segments S1a, S1b and S2a, S2b join at a point towards a central longitudinal axis 86 of the capsule. In contrast, a semi-circular shaped crack edge C1, C2, which is part of the respective perforation opening O1, O2, is arranged pointing away from the central longitudinal axis of the capsule. Therefore, the perforation opening O1, O2 is larger and more stable flaps than that provided by the prior art piercing method.

Small strong flaps are created. This construction allows to create even well-cutted triangular-like bent flaps instead of one bigger flap.

In particular, one reason for the larger perforation opening O1, O2 is that the extracentric tip of the piercing needle first hits the capsule at a point, where the crack edge C1, C2 is located afterwards. While entering the hemispherical part of the capsule the piercing needle, the needle cuts the shell of the hemispherical regions oriented towards the axis 86 apart into the two segments S1a, S1b or S2a, S2b. By providing two separate segments, the perforation opening tends to be more stable after retraction of the needle.

The below table 3 shows results from measurements and reveals how the improvement works in terms of emitted dose. Apparently, there is a better comparative performance, meaning a higher-end dose and less variation between delivered doses DD in percent (DD%) that leaves the capsule and enters the lung.

**Table 3**

| | **DD% for Prior Art Inhaler** | **DD% for Inhaler described herein** |
|---|---|---|
| | 49,57 | 57,22 |
| | 56,59 | 58,22 |
| | 57,41 | 55, 64 |
| | | |
| | 51,10 | 59,35 |
| | 53,52 | 58,15 |
| | 52,41 | 57,75 |
| | 53,50 | 59, 65 |
| | 52, 87 | 57,76 |
| | 51,95 | 56, 87 |
| | 56, 84 | 54, 94 |
| | | |
| Mean in DD% | 53,58 | 57,56 |
| Variation in DD% | 4,2 | 1,4 |
| | | |

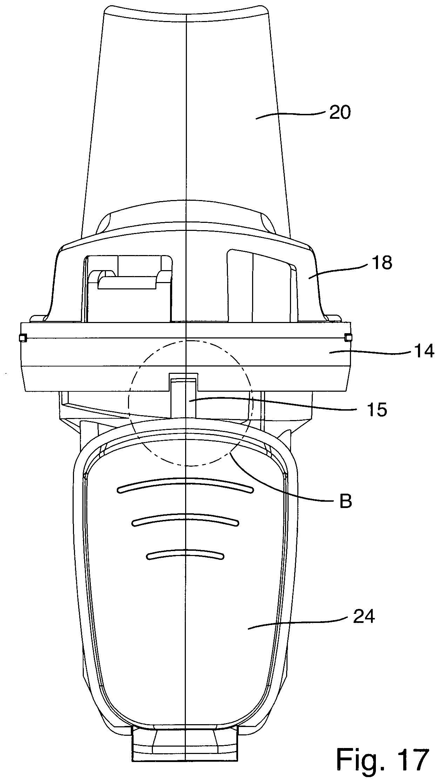

Figure 15 to 17 depict an example of the dry powder inhaler 10 without the covering body 16. The features are compatible to and combinable with the other examples of the dry powder inhaler 10 described before.

The first end 30 of each actuator button 24 is pivotally attached to the housing 12 by use of the ball joint snap-on connection 34 pivoting around the pivot axis, wherein the actuator button 4 is pivotable around a pivoting angle 36 of about 5° to 15°, preferably of about 10°.

The ball joint snap-on connection 34 comprises the hemispherical protrusions (not shown in the drawings) arranged on the housing 12 and the hemi-spherical recesses 44 (shown in Figure 15) arranged on the actuator buttons 24, wherein the protrusions and recesses extend along the pivot axis and wherein the protrusions engage the recesses.

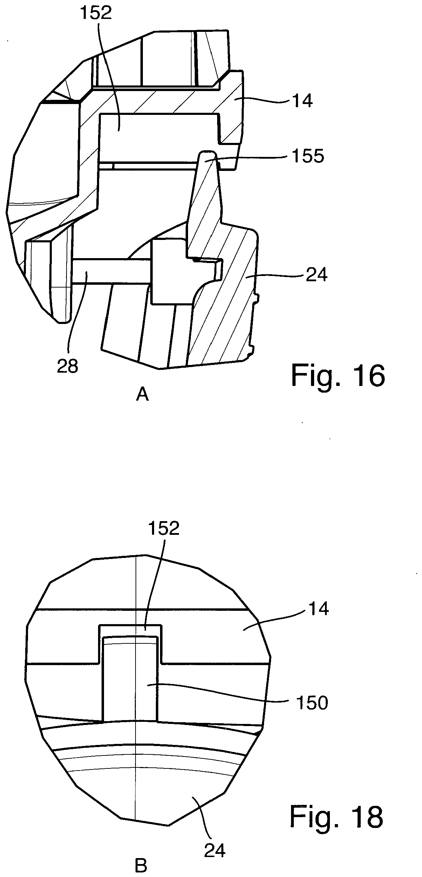

A contour 150, as shown in figure 18, or second end faces away from the connection 24. The contour 150 or second end has the shape of a fin and engages in a guiding contour 152, which is configured, for example, as a groove. The respective second end 150 moves along a circular path around the pivot axis. According to another example, the contour or second end 150 is a groove and the guiding contour 152 of the base body 14 is a fin guided in the groove. Of course, the contour does not rely on being arranged on the second or rotating end of the actuator button 24.

The contact surface of the actuator button 24, which is pressed by the user with his hands, is at least partly surrounded by an outwardly facing sealing surface 160. The sealing surface 160, when pressed outwardly by the spring 46, abuts an inwardly facing mating surface of the covering body 16, which is not shown here, thereby reducing the air supply over the edges of the actuator buttons 24. The contour or second end 150 is arranged proximal to or flush to the outwardly facing sealing surface 160 of the respective actuator button 24.

In other words, the respective actuator button 24 comprises a contour, especially the at least one guiding fin, moving along a circular path around the pivot axis, wherein the contour engages in the guiding contour 152, especially a groove, of the base body 14. Advantageously, the base body serves an additional guiding means to maintain a stable guiding of the actuator button 24 and the piercing needle 28 attached thereto.

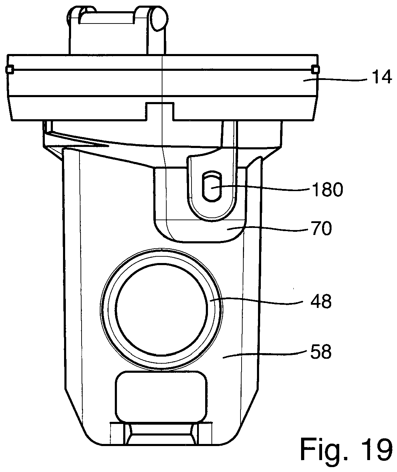

Figure 19 depicts the base body 14. The hollow needle guiding section 70 comprises a through slot 180 extending from the exterior of the capsule receptacle 56 to the interior of the capsule receptacle 56. The respective needle 28 extends through the through slot 180.

The slot 180 is larger parallel to the longitudinal axis 40 than in a perpendicular plane of the longitudinal axis 40. This allows the needle, in particular its tip, to move along a circular path towards the capsule to be opened. When contact is made with the capsule, due to the rotation of the needle tip, the capsule is pressed down into the capsule receptacle 56, held in place and its shell is perforated.

## Claims

1. A dry powder inhaler for a capsule (90) containing dry powder, the inhaler (10) comprising a base body (14) having a capsule receptacle (56), two actuator buttons (24) arranged on opposing sides of the base body (14) and two perforation needles (28), each needle (28) being fixedly connected to a respective one of the actuator buttons (24) and movable relative to guiding sections (70) of the base body (14) towards each other from a normal position to a perforation position along an actuation direction (26) to perforate a capsule (90) arranged in the capsule receptacle (56), wherein the capsule receptacle (56) is arranged in an inclined angle (68) within the range of about 40° to about 50° with respect to the actuation direction (26), **characterized in that** at least one of or both of the piercing needles (28) comprise an extracentric tip (202) located on an imaginary cylindrical barrel of said needle (28) and the rotational position of said needle (28) determines that the needle tip (202) is in a tip position which faces a longitudinal axis (92) of the other piercing needle (28) to ensure that in use the needle tip (202) of said needle (28) hits the capsule shell substantially perpendicularly.

2. The dry powder inhaler according to claim 1, wherein the extracentric tip (202) of at least one of the perforation needles (28) passes an imaginary plane (212), which is tangential to an associated curved section (204) of the capsule receptacle (56), with an angle between 90° and 83°, in particular between 90° and 85°, in particular between 90° and 88°, when being moved between the normal position to the perforation position along the actuation direction (26).

3. The dry powder inhaler according to one of the claims 1 to 2, wherein a central longitudinal axis (204) of the guiding section (210) enters a distal portion (204D) of the curved section (204) in the capsule receptacle (56).

4. The dry powder inhaler according to one of the claims 1 to 3, wherein at least one of the guiding sections (70) and the associated needle (28) are arranged such that the tip (202) of at least one of the needles (28) enters the capsule receptacle (56) in a distal portion (204D) of a curved section (204) of the capsule receptacle (56).

5. The dry powder inhaler according to one of the claims 1 to 4, wherein the guiding sections (70) of the base body (14) extend parallel in parallel planes.

6. The dry powder inhaler according to one of the claims 1 to 5, the inhaler (10) comprising a capsule chamber (54) and a covering body (16) connected to the base body (14), and comprising a mouthpiece (18) covering the capsule chamber (54) and operable for sucking air through the capsule chamber (54), wherein the mouthpiece (18) has an inhaling inlet (22) fluidly connected to the capsule chamber (54).

7. The dry powder inhaler (10) according to one of the claims 1 to 6, wherein the capsule chamber (54) is circular cylindrical and comprises an at least partly hemi-spherical end-section (66) having the capsule receptacle (56).

8. The dry powder inhaler (10) according to one of the claims 1 to 7, wherein the mouthpiece (18) comprises a cylindrical protrusion (91) configured to extend at least partially into the capsule chamber (54).

9. The dry powder inhaler of claim 7 and 8, wherein the cylindrical protrusion (91) of the mouthpiece (18) extends into the capsule chamber (54) wherein a lateral surface (93) of the cylindrical protrusion is configured to sealingly abut on an inside surface (95) of the capsule chamber (54).

10. The dry powder inhaler (10) according to one of the claims 1 to 9, wherein the base body (14) comprises at least one base body duct (87) overlapping with and in fluid connection with the mouthpiece air inlet (85), wherein the base body duct (87) is fluidly connected to the intermediate air chamber (81) and ends in the intermediate air chamber (81).

11. The dry powder inhaler (10) according to one of the claims 1 to 10, wherein the base body (14) comprises a cylindrical shoulder (52) and a base body plate (50) arranged perpendicular to the cylindrical shoulder (52).

12. The dry powder inhaler (10) according to one of the claims 1 to 11, wherein the capsule chamber (54) is attached to the base body plate (50) and opens into the base body plate (50).

13. The dry powder inhaler (10) according to one of the preceding claims, wherein the extracentric tip (202) of at least one of the perforation needles (28) hits the capsule at a point at which a later crack edge (C1, C2) of a perforation opening (O1, O2) is formed, and wherein the at least one perforation needle (28), upon further penetration into a respective hemispherical part of the capsule (90), breaks a shell of the respective hemispherical part into at least two segments (S1a, S1b; S2a, S2b).

14. The dry powder inhaler (10) of any of the preceding claims, wherein respective distal part of the perforation needle (28) is bevel-like and/or lancet-like.

15. The dry powder inhaler (10) of any of the preceding claims, wherein the respective actuator button (24) is pivotable around a pivot axis with a pivoting angle (36), for example of about 5° to 15°, preferably of about 10°, wherein the respective actuator button (24) comprises a countour, especially at least one guiding fin, moving along a circular path around the pivot axis, and wherein the countour engages in a guiding countour (152), especially a groove, of the base body (14).

16. The dry powder inhaler (10) according to the preceding claim, wherein the actuator button (24) comprises a sealing surface (160) that surrounds at least partly an outward actuator surface, and wherein the contour that engages in the guiding contour (150) of the base body (14) is arranged proximal to or flush to the outwardly facing sealing surface (160) of the respective actuator button (24).

## Patentansprüche

1. Trockenpulverinhalator für eine Kapsel (90), die Trockenpulver enthält, der Inhalator (10) umfassend einen Grundkörper (14), der eine Kapselaufnahme (56), zwei Betätigungsknöpfe (24), die auf gegenüberliegenden Seiten des Grundkörpers (14) angeordnet sind, und zwei Perforationsnadeln (28) aufweist, wobei jede Nadel (28) mit einem jeweiligen einen der Betätigungsknöpfe (24) fest verbunden ist und relativ zu Führungsbereichen (70) des Grundkörpers (14) aus einer Normalposition in eine Perforationsposition entlang einer Betätigungsrichtung (26) aufeinander zu bewegbar ist, um eine Kapsel (90), die in der Kapselaufnahme (56) angeordnet ist, zu perforieren, wobei die Kapselaufnahme (56) in einem geneigten Winkel (68) innerhalb des Bereichs von etwa 40° bis etwa 50° hinsichtlich der Betätigungsrichtung (26) angeordnet ist, **dadurch gekennzeichnet, dass** mindestens eine oder beide der Durchstechnadeln (28) eine extrazentrische Spitze (202), die sich auf einem imaginären zylindrischen Schaft der Nadel (28) befindet, umfassen und die Drehposition der Nadel (28) bestimmt, dass die Nadelspitze (202) in einer Spitzenposition ist, die einer Längsachse (92) der anderen Durchstechnadel (28) zugewandt ist, um sicherzustellen, dass in Verwendung die Nadelspitze (202) der Nadel (28) im Wesentlichen senkrecht auf die Kapselhülle trifft.

2. Trockenpulverinhalator nach Anspruch 1, wobei die extrazentrische Spitze (202) mindestens einer der Perforationsnadeln (28) eine imaginäre Ebene (212) passiert, die zu einem zugehörigen gekrümmten Bereich (204) der Kapselaufnahme (56) mit einem Winkel zwischen 90° und 83°, insbesondere zwischen 90° und 85°, insbesondere zwischen 90° und 88°, tangential ist, wenn sie zwischen der Normalposition in die Perforationsposition entlang der Betätigungsrichtung (26) bewegt wird.

3. Trockenpulverinhalator nach einem der Ansprüche 1 bis 2, wobei eine zentrale Längsachse (204) des Führungsbereichs (210) in einen distalen Abschnitt (204D) des gekrümmten Bereichs (204) in der Kapselaufnahme (56) eintritt.

4. Trockenpulverinhalator nach einem der Ansprüche 1 bis 3, wobei mindestens einer der Führungsbereiche (70) und die zugehörige Nadel (28) angeordnet sind, derart, dass die Spitze (202) mindestens einer der Nadeln (28) in die Kapselaufnahme (56) in einem distalen Abschnitt (204D) eines gekrümmten Bereichs (204) der Kapselaufnahme (56) eintritt.

5. Trockenpulverinhalator nach einem der Ansprüche 1 bis 4, wobei sich die Führungsbereiche (70) des Grundkörpers (14) in parallelen Ebenen parallel erstrecken.

6. Trockenpulverinhalator nach einem der Ansprüche 1 bis 5, der Inhalator (10) umfassend eine Kapselkammer (54) und einen Abdeckkörper (16), der mit dem Grundkörper (14) verbunden ist, und umfassend ein Mundstück (18), das die Kapselkammer (54) abdeckt und zum Ansaugen von Luft durch die Kapselkammer (54) betriebsfähig ist, wobei das Mundstück (18) einen Inhalationseinlass (22), der mit der Kapselkammer (54) fluidisch verbunden ist, aufweist.

7. Trockenpulverinhalator (10) nach einem der Ansprüche 1 bis 6, wobei die Kapselkammer (54) kreiszylindrisch ist und einen mindestens teilweise halbkugelförmigen Endbereich (66), der die Kapselaufnahme (56) aufweist, umfasst.

8. Trockenpulverinhalator (10) nach einem der Ansprüche 1 bis 7, wobei das Mundstück (18) einen zylindrischen Vorsprung (91), der konfiguriert ist, um sich mindestens teilweise in die Kapselkammer (54) zu erstrecken, umfasst.

9. Trockenpulverinhalator nach Anspruch 7 und 8, wobei sich der zylindrische Vorsprung (91) des Mundstücks (18) in die Kapselkammer (54) erstreckt, wobei eine Seitenoberfläche (93) des zylindrischen Vorsprungs konfiguriert ist, um an einer Innenoberfläche (95) der Kapselkammer (54) abdichtend anzuliegen.

10. Trockenpulverinhalator (10) nach einem der Ansprüche 1 bis 9, wobei der Grundkörper (14) mindestens einen Grundkörperkanal (87), der sich mit dem Mundstücklufteinlass (85) überlappt und in Fluidverbindung mit diesem steht, umfasst, wobei der Grundkörperkanal (87) mit der Zwischenluftkammer (81) fluidisch verbunden ist und in der Zwischenluftkammer (81) endet.

11. Trockenpulverinhalator (10) nach einem der Ansprüche 1 bis 10, wobei der Grundkörper (14) einen zylindrischen Ansatz (52) und eine Grundkörperplatte (50), die zu dem zylindrischen Ansatz (52) senkrecht angeordnet ist, umfasst.

12. Trockenpulverinhalator (10) nach einem der Ansprüche 1 bis 11, wobei die Kapselkammer (54) an der Grundkörperplatte (50) angebracht ist und in die Grundkörperplatte (50) mündet.

13. Trockenpulverinhalator (10) nach einem der vorstehenden Ansprüche, wobei die extrazentrische Spitze (202) mindestens einer der Perforationsnadeln (28) die Kapsel an einem Punkt, an dem eine spätere Risskante(C1, C2) einer Perforationsöffnung (O1, O2) ausgebildet ist, trifft, und wobei die mindestens eine Perforationsnadel (28), bei weiterer Eindringung in einen jeweiligen halbkugelförmigen Teil der Kapsel (90), eine Hülle des jeweiligen halbkugelförmigen Teils in mindestens zwei Segmente (S1a, S1b ; S2a, S2b) bricht.

14. Trockenpulverinhalator (10) nach einem der vorstehenden Ansprüche, wobei der jeweilige distale Teil der Perforationsnadel (28) schräg- und/oder lanzettenartig ist.

15. Trockenpulverinhalator (10) nach einem der vorstehenden Ansprüche, wobei der jeweilige Betätigungsknopf (24) um eine Schwenkachse mit einem Schwenkwinkel (36) von beispielsweise etwa 5° bis 15°, vorzugsweise von etwa 10°, schwenkbar ist, wobei der jeweilige Betätigungsknopf (24) eine Kontur, besonders mindestens eine Führungsrippe, die sich einen kreisförmigen Pfad um die Schwenkachse entlang bewegt, umfasst, und wobei die Kontur in eine Führungskontur (152), besonders eine Nut, des Grundkörpers (14) eingreift.

16. Trockenpulverinhalator (10) nach dem vorstehenden Anspruch, wobei der Betätigungsknopf (24) eine Abdichtungsoberfläche (160), die eine nach außen weisende Betätigungsoberfläche mindestens teilweise umgibt, umfasst, und wobei die Kontur, die in die Führungskontur (150) des Grundkörpers (14) eingreift, zu der nach außen weisenden Abdichtungsoberfläche (160) des jeweiligen Betätigungsknopfs (24) proximal oder bündig angeordnet ist.

## Revendications

1. Inhalateur de poudre sèche pour une capsule (90) contenant de la poudre sèche, l'inhalateur (10) comprenant un corps de base (14) présentant un réceptacle de capsule (56), deux boutons d'actionneur (24) agencés sur des côtés opposés du corps de base (14) et deux aiguilles de perforation (28), chaque aiguille (28) étant reliée de manière fixe à l'un des boutons d'actionneur (24) et pouvant être déplacée par rapport aux sections de guidage (70) du corps de base (14) d'une position normale à une position de perforation le long d'une direction d'actionnement (26) pour perforer une capsule (90) agencée dans le réceptacle de capsule (56), dans lequel le réceptacle de capsule (56) est agencé selon un angle incliné (68) compris entre environ 40° et environ 50° par rapport à la direction d'actionnement (26), **caractérisé en ce qu'**au moins l'une parmi les aiguilles de perçage (28) ou les deux comprennent une pointe extracentrique (202) située sur un barillet cylindre imaginaire de ladite aiguille (28) et que la position de rotation de ladite aiguille (28) détermine que la pointe de l'aiguille (202) est dans une position de pointe qui fait face à un axe longitudinal (92) de l'autre aiguille de perçage (28) afin de garantir que lors de l'utilisation, la pointe de l'aiguille (202) de ladite aiguille (28) frappe l'enveloppe de la capsule de manière sensiblement perpendiculaire.

2. Inhalateur de poudre sèche selon la revendication 1, dans lequel la pointe extracentrique (202) d'au moins une parmi les aiguilles de perforation (28) passe un plan imaginaire (212), qui est tangent à une section courbée associée (204) du réceptacle de capsule (56), avec un angle compris entre 90° et 83°, en particulier entre 90° et 85°, en particulier entre 90° et 88°, lors du passage de la position normale à la position de perforation le long de la direction d'actionnement (26).

3. Inhalateur de poudre sèche selon l'une des revendications 1 à 2, dans lequel un axe longitudinal central (204) de la section de guidage (210) pénètre dans une partie distale (204D) de la section courbée (204) dans le réceptacle de capsule (56).

4. Inhalateur de poudre sèche selon l'une des revendications 1 à 3, dans lequel au moins une parmi les sections de guidage (70) et l'aiguille associée (28) sont agencées de telle sorte que l'extrémité (202) d'au moins une parmi les aiguilles (28) pénètre dans le réceptacle de capsule (56) dans une partie distale (204D) d'une section courbée (204) du réceptacle de capsule (56).

5. Inhalateur de poudre sèche selon l'une des revendications 1 à 4, dans lequel les sections de guidage (70) du corps de base (14) s'étendent parallèlement dans des plans parallèles.

6. Inhalateur de poudre sèche selon l'une des revendications 1 à 5, l'inhalateur (10) comprenant une chambre de capsule (54) et un corps de couverture (16) relié au corps de base (14), et comprenant un embout buccal (18) recouvrant la chambre de capsule (54) et pouvant être utilisé pour aspirer l'air à travers la chambre de capsule (54), dans lequel l'embout buccal (18) présente une entrée d'inhalation (22) reliée de manière fluidique à la chambre de la capsule (54).

7. Inhalateur de poudre sèche (10) selon l'une des revendications 1 à 6, dans lequel la chambre de la capsule (54) est cylindrique circulaire et comprend une section terminale (66) au moins partiellement hémisphérique présentant le réceptacle de capsule (56).

8. Inhalateur de poudre sèche (10) selon l'une des revendications 1 à 7, dans lequel l'embout buccal (18) comprend une protubérance cylindrique (91) conçue pour s'étendre au moins partiellement dans la chambre de la capsule (54).

9. Inhalateur de poudre sèche des revendications 7 et 8, dans lequel la protubérance cylindrique (91) de l'embout buccal (18) s'étend dans la chambre de la capsule (54), dans lequel une surface latérale (93) de la protubérance cylindrique est conçue pour s'appuyer de manière étanche sur une surface intérieure (95) de la chambre de la capsule (54).

10. Inhalateur de poudre sèche (10) selon l'une des revendications 1 à 9, dans lequel le corps de base (14) comprend au moins un conduit de corps de base (87) chevauchant l'entrée d'air de l'embout buccal (85) et en raccord fluidique avec celle-ci, dans lequel le conduit de corps de base (87) est relié de manière fluidique à la chambre d'air intermédiaire (81) et se termine dans la chambre d'air intermédiaire (81).

11. Inhalateur de poudre sèche (10) selon l'une des revendications 1 à 10, dans lequel le corps de base (14) comprend un épaulement cylindrique (52) et une plaque de corps de base (50) agencée perpendiculairement à l'épaulement cylindrique (52).

12. Inhalateur de poudre sèche (10) selon l'une des revendications 1 à 11, dans lequel la chambre de la capsule (54) est fixée à la plaque de corps de base (50) et s'ouvre dans la plaque de corps de base (50).

13. Inhalateur de poudre sèche (10) selon l'une des revendications précédentes, dans lequel la pointe extracentrique (202) d'au moins une parmi les aiguilles de perforation (28) frappe la capsule à un point où un bord de fissure ultérieur (C1, C2) d'une ouverture de perforation (O1, O2) est formé, et dans lequel au moins une aiguille de perforation (28), lors de la pénétration supplémentaire dans une partie hémisphérique respective de la capsule (90), brise une enveloppe de la partie hémisphérique respective en au moins deux segments (S1a, S1b; S2a, S2b).

14. Inhalateur de poudre sèche (10) selon l'une quelconque des revendications précédentes, dans lequel la partie distale respective de l'aiguille de perforation (28) est en forme de biseau et/ou de lancette.

15. Inhalateur de poudre sèche (10) selon l'une quelconque des revendications précédentes, dans lequel le bouton d'actionneur respectif (24) peut pivoter autour d'un axe de pivotement avec un angle de pivotement (36), par exemple d'environ 5° à 15°, de préférence d'environ 10°, dans lequel le bouton d'actionneur respectif (24) comprend un contour, en particulier au moins une ailette de guidage, se déplaçant le long d'un chemin circulaire autour de l'axe de pivotement, et dans lequel le contour entre en prise dans un contour de guidage (152), en particulier une rainure, du corps de base (14).

16. Inhalateur de poudre sèche (10) selon la revendication précédente, dans lequel le bouton d'actionneur (24) comprend une surface d'étanchéité (160) qui entoure au moins partiellement une surface d'actionneur extérieure, et dans lequel le contour qui entre en prise dans le contour de guidage (150) du corps de base (14) est agencé proche ou au ras de la surface d'étanchéité tournée vers l'extérieur (160) du bouton d'actionneur respectif (24).
